(19) Europäisches Patentamt
European Patent Office
Office européen des brevets

(11) Veröffentlichungsnummer: **0 094 909**
**B1**

# (12) EUROPÄISCHE PATENTSCHRIFT

(45) Veröffentlichungstag der Patentschrift: **05.11.86**

(51) Int. Cl.⁴: **C 07 D 307/86,** A 01 N 47/24

(21) Anmeldenummer: **83810195.4**

(22) Anmeldetag: **09.05.83**

## (54) N-Alkyliden-imino-oxicarbonyl-N-methyl-(2,2-dimethyl-2,3-dihydrobenzofuranyl(7))-carbamate, Verfahren zu ihrer Herstellung und ihre Verwendung in der Schädlingsbekämpfung.

(30) Priorität: **17.05.82 CH 3053/82**
**18.02.83 CH 902/83**

(43) Veröffentlichungstag der Anmeldung:
**23.11.83 Patentblatt 83/47**

(45) Bekanntmachung des Hinweises auf die Patenterteilung:
**05.11.86 Patentblatt 86/45**

(84) Benannte Vertragsstaaten:
**AT BE CH DE FR IT LI NL**

(56) Entgegenhaltungen:
**EP-A-0 005 246**
**EP-A-0 022 498**

**K.H. BUECHEL, Pflanzenschutz- und Schädlingsbekämpfung, G. Thieme Verlag, Stuttgart, 1977, Seiten 60,66**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Drabek, Jozef, Dr., Benkenstrasse 12, CH-4104 Oberwil (CH)**

**EP 0 094 909 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft N-Alkyliden-imino-oxicarbonyl-N-methyl-[2,2-dimethyl-2,3-dihydrobenzofuranyl(7)]-carbamate. Verfahren zu ihrer Herstellung und ihre Verwendung in der Schädlingsbekämpfung.

Die N-Alkyliden-imino-oxicarbonyl-N-methyl-[2,2-dimethyl-2,3-dibydro-benzofuranyl(7)]-carbamate haben die Formel

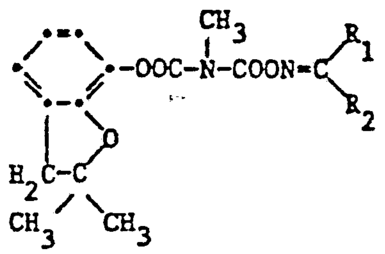

(I),

worin $R_1$ und $R_2$ unabhängig voneinander eine $C_1$-$C_{10}$-Alkyl- oder zusammen eine $C_2$-$C_9$-Alkylengruppe bedeuten.

Die Alkylgruppen bei $R_1$ und $R_2$ können geradkettig oder verzweigt sein. Beispiele solcher Gruppen sind u.a: Methyl, Aethyl, Propyl, Isopropyl, n-, i-, sek.-, tert.-Butyl, n-Pentyl, n-Hexyl, n-Heptyl, n-Octyl, n-Nonyl, n-Decyl.

Alkylengruppen bei $R_1$ und $R_2$ können geradkettig oder verzweigt sein. Beispiele solcher Gruppen sind u.a. Dimethylen, Trimethylen, Tetramethylen. Pentamethylen, Nonamethylen,

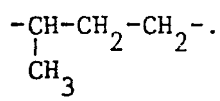

Bevorzugt sind Verbindungen der Formel I, worin $R_1$ und $R_2$ unabhängig voneinander eine $C_1$-$C_5$-Alkyl- oder zusammen eine $C_3$-$C_9$-Alky-lengruppe bedeuten. Ganz besonders bevorzugtsind aber Verbindungen der Formel I, worin $R_1$ eine $C_1$-$C_5$-Alkylgruppe, $R_2$ Methyl, Aethyl oder propyl oder $R_1$ und $R_2$ zusammen die Tetra- oder pentamethylengruppe bedeuten.

Die Verbindungen der Formel I können nach an sich bekannten Methoden z.B. wie folgt hergestellt werden:

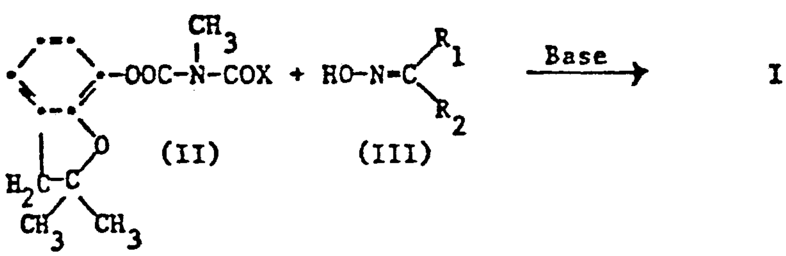


(II) (III) I

In den formeln II und III haben $R_1$ und $R_2$ die für die Formel I angegebene Bedeutung und X steht für ein Halogenatom, insbesondere für Fluor oder Chlor.

Das Verfahren kann man bei einer Reaktionstemperatur zwischen -50°C und +130°C, vorzugsweise zwischen -10°C und +100°C, bei normalem oder leicht erhöhtem Druck und in Gegenwart einer Base und gegebenenfalls eines gegenüber den Reaktionsteilnehmern inerten Lösungs- oder Verdünnungsmittels durchführen.

Als geeignete Basen für dieses Verfahren kommen insbesondere tertiäre Amine, wie Trialkylamine, Pyridine und Dialkylaniline, ferner Hydroxide, Oxide, Carbonate und Bicarbonate von Alkali- und Erdalkalimetallen sowie Alkalimetallalkoholate wie z.B. Kalium-tert.butylat oder Natrium-methylat in Betracht.

Als Lösungs- oder Verdünnungsmittel eignen sich z.B. Aether und ätherartige Verbindungen wie Diäthyläther, Di-isopropyläther, Dioxan, Tetrahydrofuran; aliphatische und aromatische Kohlenwasserstoffe, insbesondere Benzol, Toluol, Xylole; Ketone wie Aceton, Methyläthylketon und Cyclohexanon.

Die Ausgangsstoffe der Formeln II und III sind bekannt bzw. können analog bekannten Methoden hergestellt werden. Die Verbindungen der Formel I eignen sich zur Bekämpfung von Schädlingen an Tieren, Pflanzen und im Boden. Die Verbindungen der Formel I eignen sich zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von phytopathogenen Milben und Zecken der Ordnung Acarina. Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwollkulturen (z.B. Spodoptera littoralis und Heliothis virescens) und Gemüsekulturen (z.B. Leptinotarsa decemlineata und Myzus persicae) und von Bodeninsekten (z.B.

Aulacophora femoralis, Chortophila brassicae, pachnoda savignyi und Scotia ypsilon).

In diesem Zusammenhang ist hervorzuheben, dass die genannten Verbindungen sich sowohl durch eine stark ausgeprägte systemische als auch Kontakt-Wirkung gegen saugende Insekten und vor allem gegen Insekten der Familie Aphididae (wie z.B. Aphis fabae, Aphis craccivora und Myzus persicae), welche sich mit bekannten Mitteln nur schwierig bekämpfen lassen, ausseichnen. Wirkstoffe der Formel I zeigen auch eine sehr günstige Wirkung gegen Fliegen, wie z.B. Musca domestica und Mückenlarven. Ferner zeichnen sie sich durch eine breite ovizide und ovolarvizide Wirkung aus und besitzen eine wertvolle Wirkung gegen ektoparasitäre Milben und Zecken z.B. der Familien Ixodidae, Argasidae und Dermanyssidae.

In der europäischen Patentveröffentlichung Nr. 5246 wird das Ncarboxylierte Carbamat der Formel

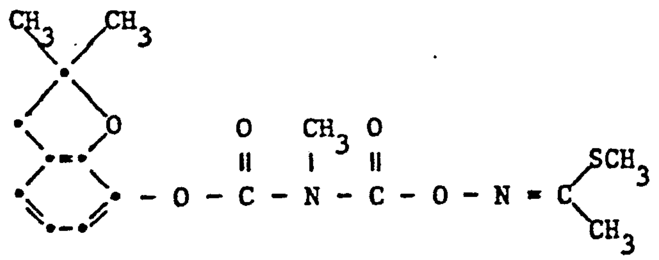

beschrieben.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes de Formel 1 nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Taleöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen

Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxid-Addukte an Polypropylenglykol, Aethylendiaminopropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Amminiumsalze, welche als N-Substituenten mindestens einen Alkylrest mit 8 bis 22 C-Atomen enthalten und als weitere Substituenten niedrige gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgender Publikation beschrieben:

"Mc Cutcheon's Detergents and Emulsifiers Annual" MC Publishing Corp., Ridgewood, New Jersey, 1979.

Die pestiziden Zubereitungen enthalten in der Regel 0,1 bis 99%, insbesondere 0,1 bis 95%, Wirkstoff der Formel I, Ibis 99,9% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

**Formulierungsbeispiele für flüssige Werkstoffe der Formel I (% = Gewichtsprozent)**

**1. Emulsions-Konzentrate**

| | a) | b) | c) |
|---|---|---|---|
| Wirkstoff | 25% | 40% | 50% |
| Ca-Dodecylbenzolsulfonat | 5% | 8% | 6% |
| Ricinusöl-polyäthylenglykoläther (36 Mol AeO) | 5% | - | - |
| Tributylphenyl-polyäthylenglykoläther (30 Mol AeO) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

**2. Lösungen**

| | a) | b) | c) | d) |
|---|---|---|---|---|
| Wirkstoff | 80% | 10% | 5% | 95% |
| Aethylenglykol-monomethyläther | 20% | - | - | - |
| Polyäthylenglykol MG 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

Die Lösungen sind zur Anwendung in Form kleinster Tropfen geeignet.

**3. Granulate**

| | a) | b) |
|---|---|---|
| Wirkstoff | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

**4. Stäubemittel**

| | a) | b) |
|---|---|---|
| Wirkstoff | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

**Formulierungsbeispiele für feste Wirkstoffe der Formel I (% = Gewichtsprozent)**

**5. Spritzpulver**

| | a) | b) | c) |
|---|---|---|---|
| Wirkstoff | 25% | 50% | 75% |
| Na-Ligninsulfonat | 5% | 5% | - |
| Na-Laurylsulfat | 3% | - | 5% |

| | a) | b) | c) |
|---|---|---|---|
| Na-Diisobutylnaphthalinsulfonat | - | 6% | 10% |
| Octylphenolpolyäthylenglykoläther (7-8 Mol AeO) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

**6. Emulsions-Konzentrat**

| | |
|---|---|
| Wirkstoff | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

**7. Stäubemittel**

| | a) | b) |
|---|---|---|
| Wirkstoff | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

**8. Extruder-Granulat**

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

**9. Umhüllungs-Granulat**

| | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

**10. Suspensions-Konzentrat**

| | |
|---|---|
| Wirkstoff | 40% |
| Aethylenglykol | 10% |
| Nonylphenolpolyäthylenglykol-äther (15 Mol AeO) | 6% |
| Na- Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Beispiel 1**

Herstellung der Verbindung der Formel

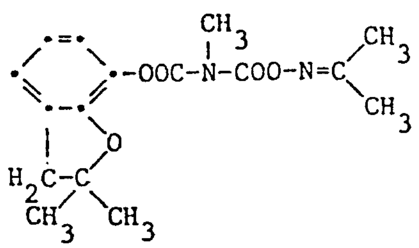

Nr. 1

Zu einer Lösung von 8,5 g Chlorcarbonyl-N-methyl-[2,2-dimethyl-2,3-dihydrobenzofuranyl(7)]-carbamat, 2,2 g Acetonoxim in 100 ml Toluol werden bei 0 bis 10° C 4,83 g Triäthylamin und 0,1 g 4-Dimethylamino-pyridin in 30 Minuten zugetropft. Nach 10-stündigem Rühren bei 20° C wird das Reaktionsgemisch je einmal mit 100 ml 0,05 N-Salzsäurelösung und 100 ml Wasser gewaschen. Nach dem Trocknen über Natriumsulfat wird das Toluol aus dem Reaktionsgemisch abdestilliert. Das Rohprodukt wird aus einem Gemisch von Hexan und Toluol (1:1) umkristallisiert.

Man erhält die Titelverbindung mit einem Schmelzpunkt von 89° C.

Auf analoge Weise werden auch folgende Verbindungen hergestellt:

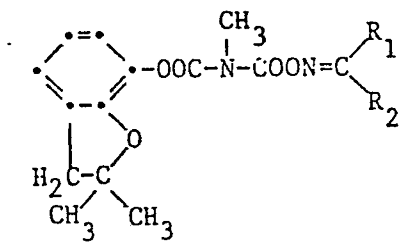

| Nr. | $R_1$ | $R_2$ | Physikalische Daten |
|---|---|---|---|
| 2 | $-CH_2-CH_2-CH_2-CH_2-$ | | $n_D^{45°} = 1,5296$ |
| 3 | $-C_4H_{9(n)}$ | $-CH_3$ | $n_D^{21°} = 1,5189$ |
| 4 | $-C_4H_{9(i)}$ | $-CH_3$ | $n_D^{22°} = 1,5181$ |
| 5 | $-CH_2-CH_2-CH(CH_3)_2$ | $-CH_3$ | $n_D^{20°} = 1,5150$ |
| 6 | $-C_2H_5$ | $-CH_3$ | $n_D^{22°} = 1,5259$ |
| 7 | $-C_3H_{7(i)}$ | $-CH_3$ | $n_D^{20°} = 1,5202$ |
| 8 | $-(CH_2)_7\ CH_3$ | $-C_3H_{7(n)}$ | $n_D^{20} = 1,5055$ |
| 9 | $-(CH_2)_9\ CH_3$ | $-CH_3$ | $n_D^{20°} = 1,5047$ |
| 10 | $-CH_2-CH_2-CH_2-$ | | $n_D^{20} = 1,5312$ |
| 11 | $-(CH_2)_9-$ | | $n_D^{40°} = 1,5250$ |
| 12 | $-CH_2-CH_2-CH_2-CH_2-CH_2-$ | | Smp.: 95 - 100°C |
| 13 | $-C_3H_{7(n)}$ | $-C_3H_{7(n)}$ | $n_D^{20°} = 1,5055$ |

**Beispiel 2: Insektizide systemtische Wirkung: Aphis craccivora**

Bewurzelte Bohnenpflanzen werden in Töpfe, welche 600ccm Erde enthalten, verpflanzt. Anschliessend werden 50 ml einar Versuchslösung, enthaltend50 ppm, 12,5 ppm bzw. 3 ppm der zuprüfenden Verbindung, direkt auf die Erde gegossen.

Nach 24 Stunden werden auf die oberirdischen Pflanzenteile Blattläuse (Aphis craccivora) gesetzt und die Pflanzen mit einem unten zugeschnürten Plastikzylinder überstülpt, um die Läuse vor einer eventuellen Kontakt- oder Gaswirkung der Testsubstanz zu schützen.

Die Auswertung der erzielten Abtötung erfolgt 48 Stunden nach Versuchsbeginn. Pro Konzentrationsdosis Testsubstanz werden zwei pflanzen, je eine in einem separaten Topf, verwendet. Der Versuch wird bei 25°C und 70% relativer Luftfeuchtigkeit durchgeführt.

Die Verbindungen gemäss Beispiel 1 haben die in der folgenden Tabelle angegebenen Wirkungen gegen Aphis craccivora.

**Biologische Versuchsergebnisse**

In der folgenden Tabelle sind Versuchsergebnisse auf der Basis des vorstehenden Beispiels aufgeführt, und zwar mit folgendem Bewertungsindex in Bezug auf die prozentuale Abtötung der Schädlinge:

A: 70-100% Abtötung bei 3 ppm Wirkstoffkonzentration

B: 70-100% Abtötung bei 12,5 ppm Wirkstoffkonzentration

C: 70-100% Abtötung bei 50 ppm Wirkstoffkonzentration

| Verbindung Nr. | Wirksamkeit gegen Aphis craccivora |
|---|---|
| 1 | A |
| 2 | B |
| 3 | B |
| 4 | A |
| 5 | A |
| 6 | A |
| 7 | B |
| 8 | A |
| 9 | B |
| 10 | B |
| 11 | B |
| 12 | A |
| 13 | B |

**Patentansprüche**

für die Vertragsstaaten BE, CH, DE, FR, IT, LI, NL.

1. N-Alkyliden-imino-oxicarbonyl-N-methyl-[2,2-dimethyl-2,3-dihydro-benzofuranyl(7)]-carbamate der Formel

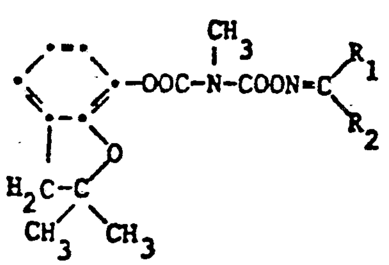,

worin $R_1$ und $R_2$ unabhängig voneinander eine $C_1$-$C_{10}$-Alkyl- oder zusammen eine $C_2$-$C_9$-Alkylengruppe bedeuten.

2. Eine Verbindung gemäss Anspruch 1, worin $R_1$ und $R_2$ unabhängig voneinander eine $C_1$-$C_5$-Alkyl- oder

zusammen eine $C_3$-$C_9$-Alkylengruppe bedeuten.

3. Eine Verbindung gemäss Anspruch 2, worin $R_1$ eine $C_1$-$C_5$-Alkyl-gruppe, $R_2$ Methyl, Aethyl oder Propyl oder $R_1$ und $R_2$ zusammen die Tetra- oder Pentamethylengruppe bedeuten.

4. Die Verbindung gemäss Anspruch 3 der Formel

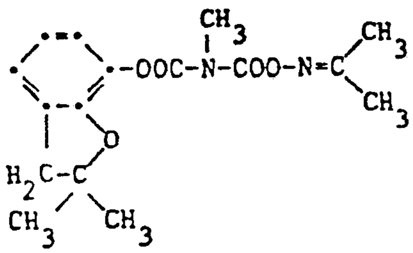

.

5. Die Verbindung gemäss Anspruch 3 der Formel

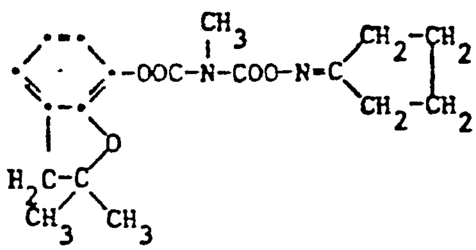

.

6. Die Verbindung gemäss Anspruch 3 der Formel

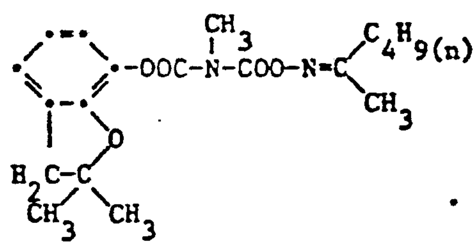

.

7. Die Verbindung gemäss Anspruch 3 der Formel

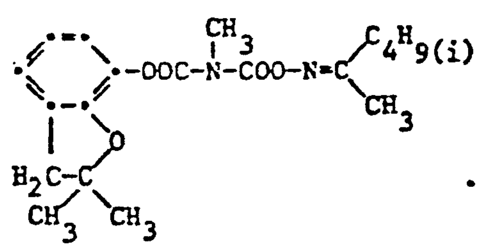

.

8. Die Verbindung gemäss Anspruch 3 der Formel

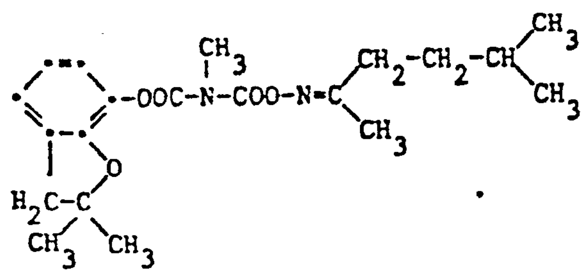

9. Die Verbindung gemäss Anspruch 3 der Formel

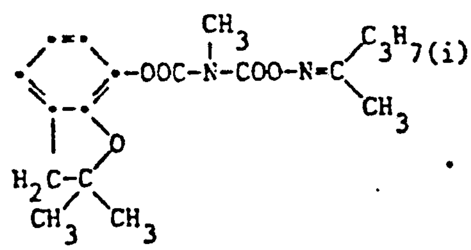

10. Die Verbindung gemäss Anspruch 3 der Formel

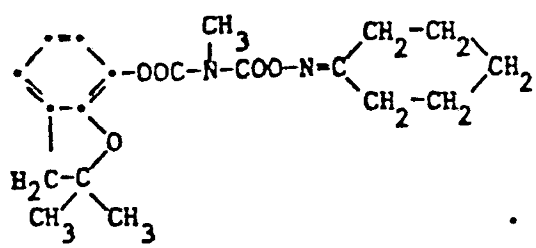

11. Die Verbindung gemäss Anspruch 1 der Formel

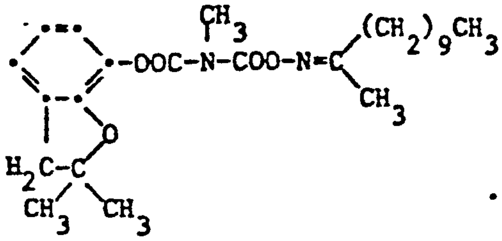

12. Die Verbindung gemäss Anspruch 3 der Formel

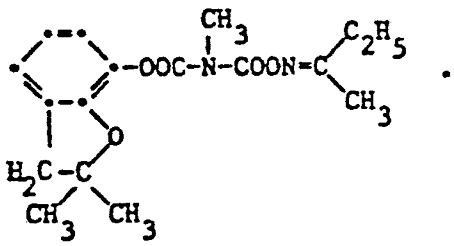

13. Die Verbindung gemäss Anspruch 3 der Formel

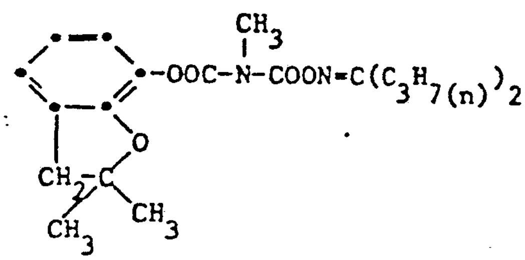

14. Die Verbindung gemäss Anspruch 2 der Formel

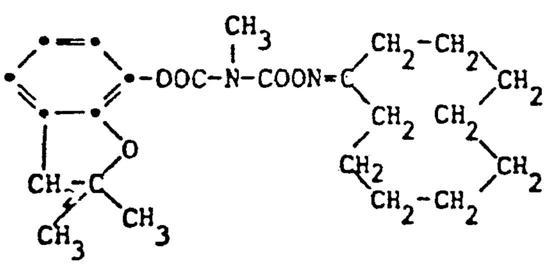

15. Die Verbindung gemäss Anspruch 2 der Formel

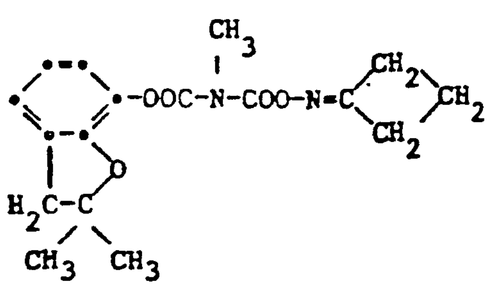

16. Verfahren zur Herstellung von Verbindungen gemäss Anspruch 1, dadurch gekennnzeichnet, dass man in Gegenwart einer Base eine Verbindung der Formel

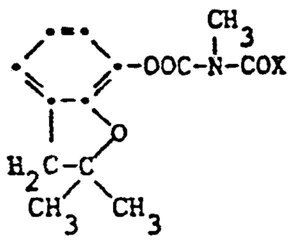

mit einer Verbindung der Formel

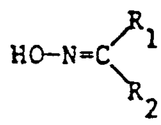

umsetzt, worin $R_1$ und $R_2$ die im Anspruch 1 angegebene Bedeutung haben und X für ein Halogenatom steht.

17. Ein Schädlingsbekämpfungsmittel, welches als aktive Komponente eine Verbindung gemäss Anspruch 1 anthält.

18. Die Verwendung einer Verbindung gemäss Anspruch 1 zur Bekämpfung von Schädlingen an Tieren, Pflanzen und im Boden.

19. Die Verwendung gemäss Anspruch 18 zur Bekämpfung von Insekten und Vertretern der Ordnung Akarina.

20. Verfahren zur Bekämpfung von Schädlingen an Tieren, pflanzen und im Boden, dadurch gekennzeichnet, dass man eine Verbindung gemäss Anspruch 1 auf die Tiere, die Pflanzen oder auf den Boden appliziert.

**Patentansprüche**

für den Vertragsstaat AT

1. Ein Schädlingsbekämpfungsmittel, welches neben einem festen oder flüssigen Zusatzstoff als aktive Komponente ein N-Alkyliden-imino-oxicarbonyl-N-methyl-[2,2-dimethyl-2,3-dihydrobenzofuranyl(7)])-carbamat der Formel

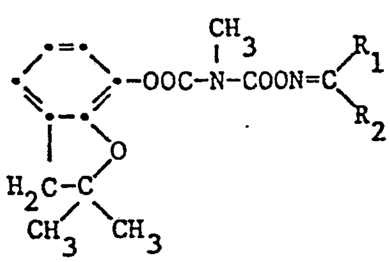

enthält,worin

$R_1$ und $R_2$ unabhängig voneinander eine $C_1$-$C_{10}$—Alkyl- oder zusammen eine $C_2$-$C_9$-Alkylengruppe bedeuten.

2. Ein Mittel gemäss Anspruch 1, worin in der Formel der aktiven Komponente $R_1$ und $R_2$ unabhängig voneinander eine $C_1$-$C_5$-Alkyl-oder zusammen eine $C_3$-$C_9$-Alkylengruppe bedeuten.

3. Ein Mittel gemäss Anspruch 2, worin in der Formel der aktiven Komponente $R_1$ eine $C_1$-$C_5$-Alkylgruppe, $R_2$ Methyl, Aethyl oder Propyl oder $R_1$ und $R_2$ zusammen die Tetra- oder Pentamethylengruppe bedeuten.

4. Ein Mittel gemäss Anspruch 3 welches als aktive Komponente die Verbindung der Formel

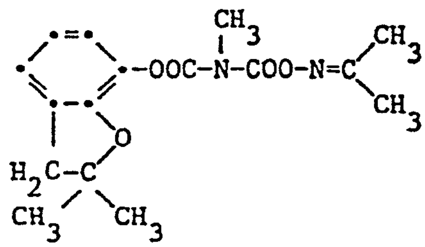

enthält.

5. Ein Mittel gemäss Anspruch 3, welches als aktive Komponente die Verbindung der Formel

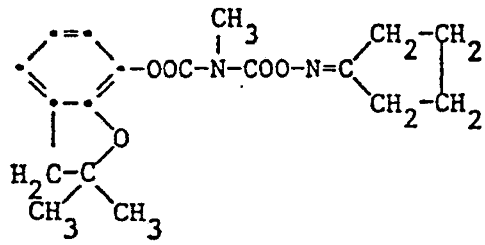

enthält.

6. Ein Mittel gemäss Anspruch 3, welches als aktive Komponente die Verbindung der Formel

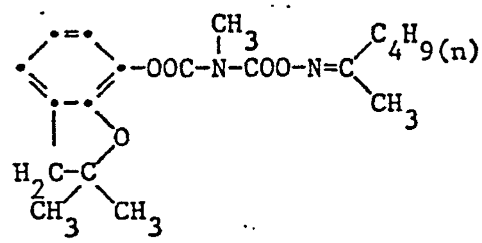

enthält.

7. Ein Mittel gemäss Anspruch 3, welches als aktive Komponente die Verbindung der Formel

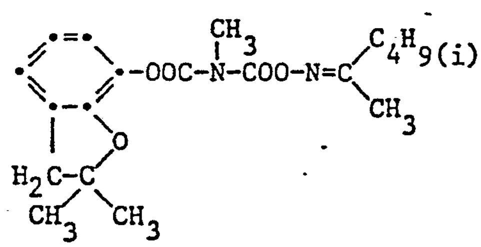

enthält.

8. Ein Mittel gemäss Anspruch 3, welches als aktive Komponente die Verbindung der Formel

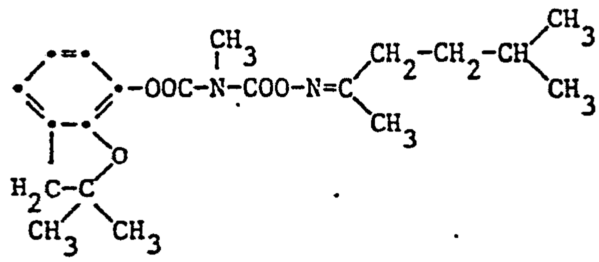

enthält.

9. Ein Mittel gemäss Anspruch 3, welches als aktive Komponente die Verbindung der Formel

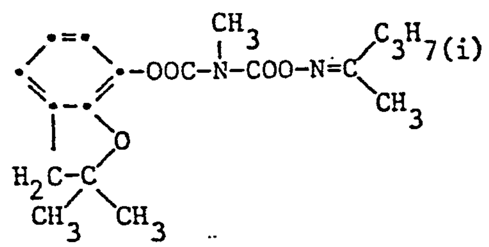

enthält.

10. Ein Mittel gemäss Anspruch 3 welches als aktive Komponente die Verbindung der Formel

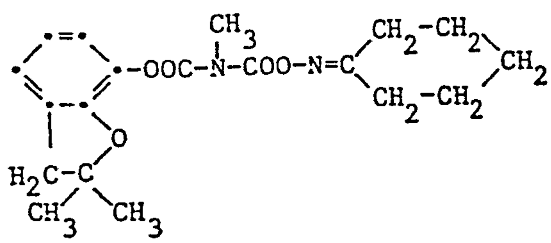

enthält.

11. Ein Mittel gemäss Anspruch 1, welches als aktive Komponente die Verbindung der Formel

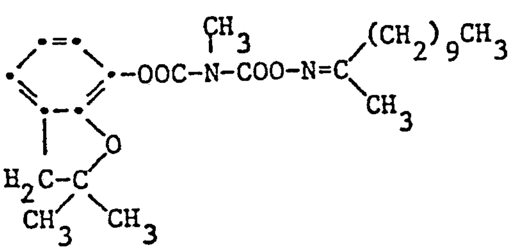

enthält.

12. Ein Mittel gemäss Anspruch 3, welches als aktive Komponente die Verbindung der Formel

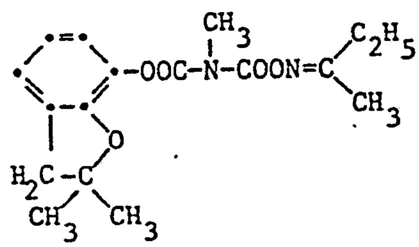

enthält.

13. Ein Mittel gemäss Anspruch 3, welches als aktive Komponente die Verbindung der Formel

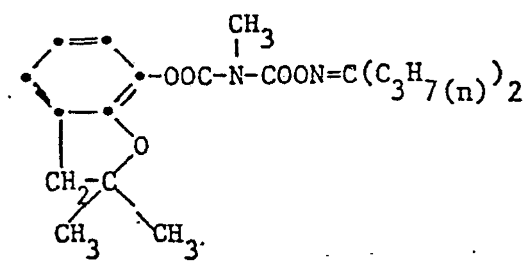

enthält.

14. Ein Mittel gemäss Anspruch 2, welches als aktive Komponente eine Verbindung der Formel

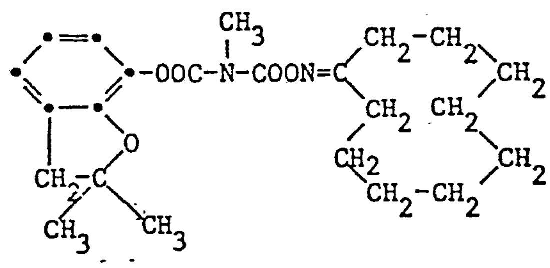

enthält.

15. Ein Mittel gemäss Anspruch 2, welches als aktive Komponente eine Verbindung der Formel

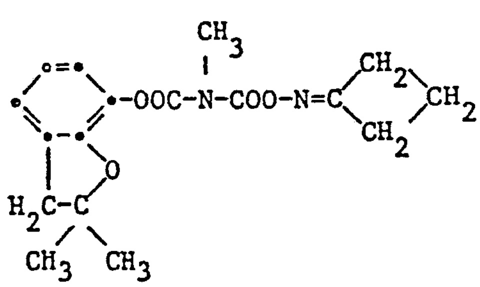

enthält.

16. Verfahren zur Herstellung von Verbindungen der Formel I gemäss Anspruch 1, dadurch gekennzeichnet, dass man in Gegenwart einer Base eine Verbindung der Formel

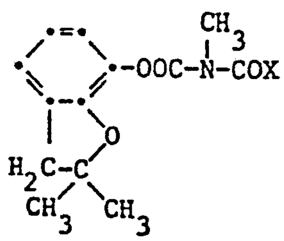

mit einer Verbindung der Formel

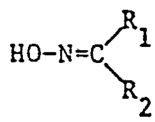

umsetzt, worin $R_1$ und $R_2$ die im Anspruch 1 angegebene Bedeutung haben und X für ein Halogenatom steht.

17. Die Verwendung eines Mittels gemäss Anspruch 1 zur Bekämpfung von Schädlingen an Tieren, Pflanzen und im Boden.

18. Die Verwendung gemäss Anspruch 17 zur Bekämpfung von Insekten und Vertretern der Ordnung Akarina.

19. Verfahren zur Bekämpfung von Schädlingen an Tieren, pflanzen und im Boden, dadurch gekennzeichnet, dass man ein Mittel gemäss Anspruch 1 auf die Tiere, die Pflanzen oder auf den Boden appliziert.

**Claims**

for the Contracting States: BE, CH, DE, FR, IT, LI, NL.

1. An N-alkylidene-imino-oxycarbonyl-N-methyl-[2,2-dimethyl-2,3-dihydrobenzofuranyl(7)]carbamate of the formula

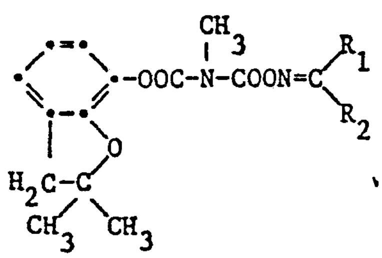

wherein $R_1$ and $R_2$ are each independently of the other a $C_1$-$C_{10}$alkyl group, or together they are a $C_2$-$C_9$alkylene group.

2. A compound according to claim 1, wherein each of $R_1$ and $R_2$ independently of the other is a $C_1$-$C_5$alkyl group, or together they are a $C_3$-$C_9$alkylene group.

3. A compound according to claim 2, wherein $R_1$ is a $C_1$-$C_5$alkylene group, $R_2$ is methyl, ethyl or propyl, or $R_1$ and $R_2$ together are the tetra- or pentamethylene group.

4. The compound according to claim 3 of the formula

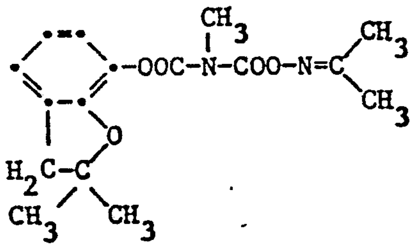

5. The compound according to claim 3 of the formula

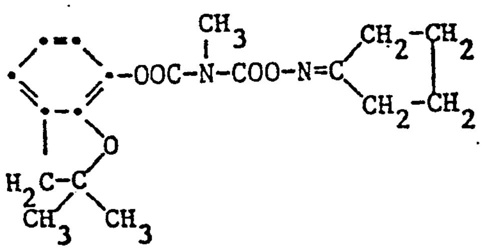

6. The compound according to claim 3 of the formula

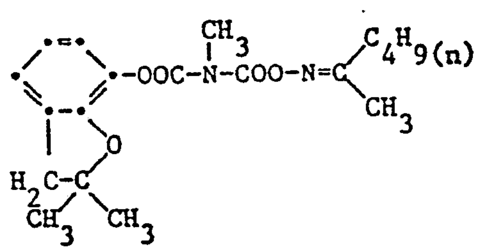

7. The compound according to claim 3 of the formula

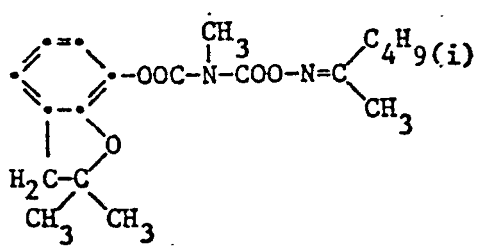

8. The compound according to claim 3 of the formula

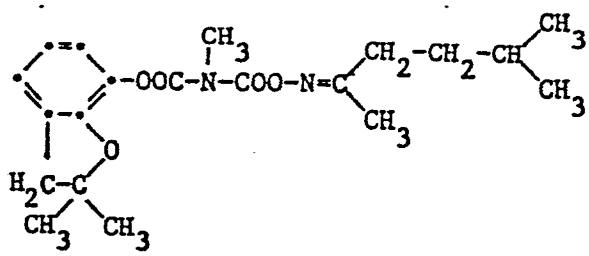

9. The compound according claim 3 of the formula

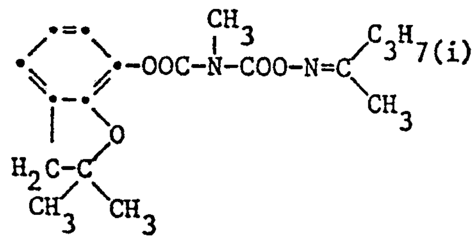

10. The compound according to claim 3 of the formula

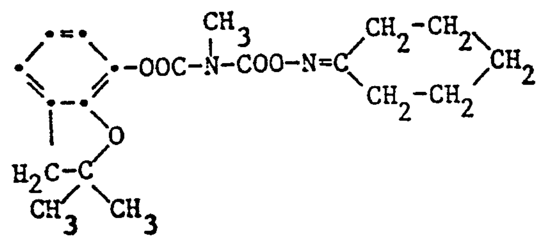

11. The compound according to claim 1 of the formula

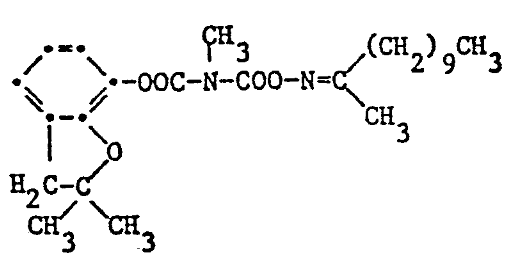

12. The compound according to claim 3 of the formula

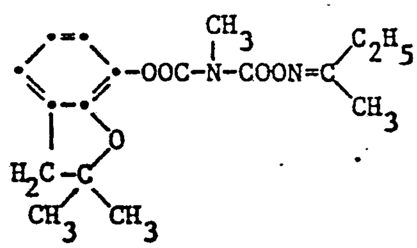

13. The compound according to claim 3 of the formula

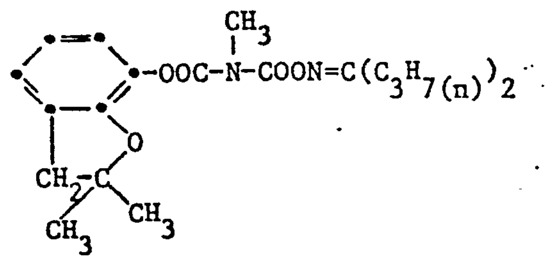

14. The compound according to claim 2 of the formula

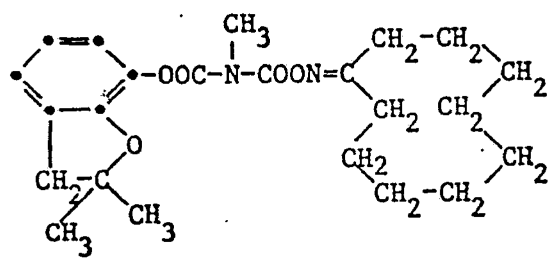

15. The compound according to claim 2 of the formula

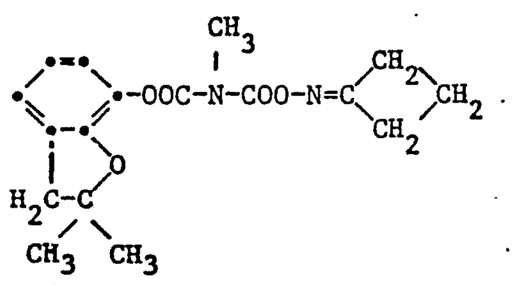

16 A process for the preparation of a compound according to claim 1, which process comprises reacting a compound of the formula

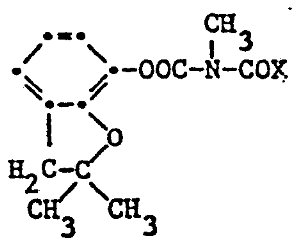

in the presence of a base, with a compound of the formula

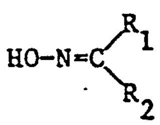

wherein $R_1$ and $R_2$ are as defined in claim 1, and X is a halogen atom.

17. A pesticidal composition which contains as active ingredient a compound according to claim 1.

18. Use of a compound according to claim 1 for controlling pests on animals and plants and in the soil.

19. Use according to claim 18 for controlling insects, and members of the order Acarina.

20. A method of controlling pests on animals and plants and in the soil, which method comprises applying to the animals, to the plants or to the soil an effective amount of a compound according to claim 1.

**Claims**

for the Contracting State: AT

1. A pesticidal composition which, in addition to a solid or liquid adjuvant, contains as active ingredient an N-alkylidene-imino-oxycarbonyl-N-methyl-[2,2-dimethyl-2,3-dihydrobenzofuranyl(7)]carbamate of the formula

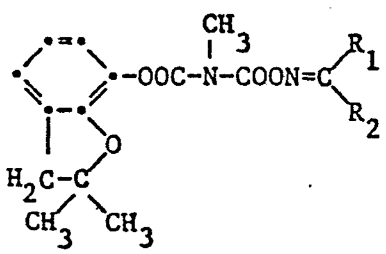

wherein $R_1$ and $R_2$ are each independently of the other a $C_1$-$C_{10}$alkyl group, or together they are a $C_2$-$C_9$alkylene group.

2. A composition according to claim 1, wherein in the formula of the active ingredient each of $R_1$ and $R_2$ independently of the other is a $C_1$-$C_5$ alkyl group, or together they are a $C_3$-$C_9$alkylene group.

3. A composition according to claim 2, wherein in the formula of the active ingredient $R_1$ is a $C_1$-$C_5$alkylene group, $R_2$ is methyl, ethyl or propyl, or $R_1$ and $R_2$ together are the tetra- or pentamethylene group.

4. A composition according to claim 3, which contains as active ingredient the compound of the formula

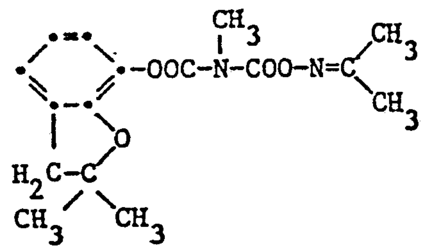

5. A composition according to claim 3, which contains as active ingredient the compound of the formula

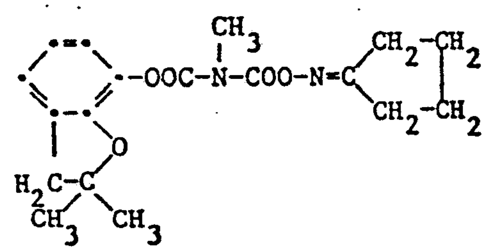

6. A composition according to claim 3, which contains as active ingredient the compound of the formula

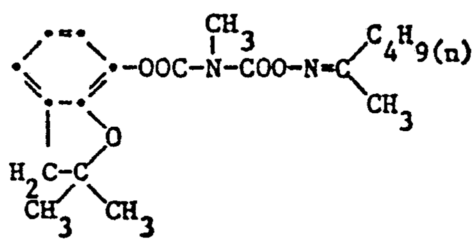

7. A composition according to claim 3, which contains as active ingredient the compound of the formula

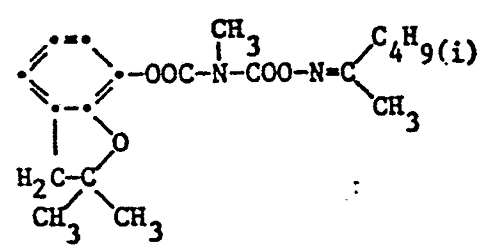

8. A composition according to claim 3, which contains as active ingredient the compound of the formula

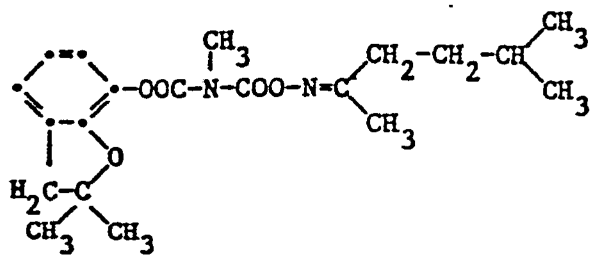

9. A composition according to claim 3, which contains as active ingredient the compound of the formula

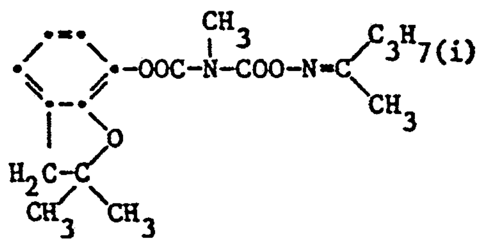

10. A composition according to claim 3, which contains as active ingredient the compound of the formula

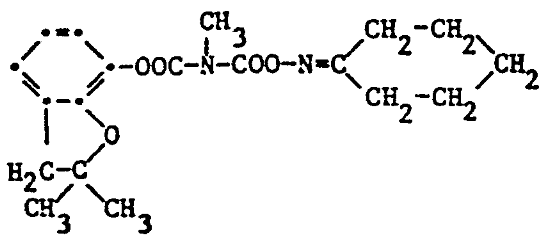

11. A composition according to claim 1, which contains as active ingredient the compound of the formula

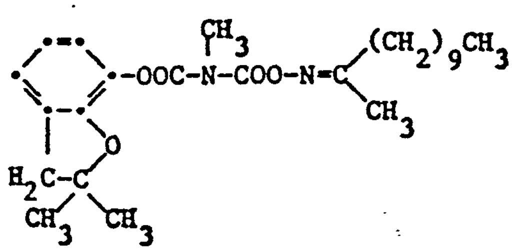

12. A composition according to claim 3, which contains as active ingredient the compound of the formula

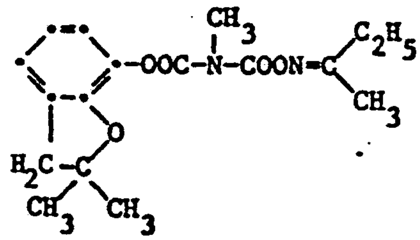

13. A composition according to claim 3, which contains as active ingredient the compound of the formula

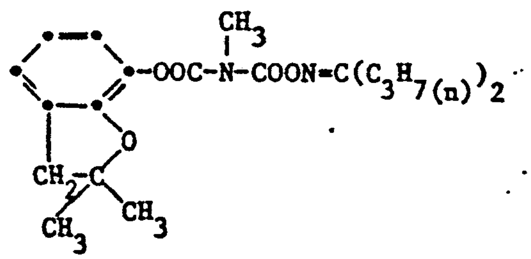

14. A composition according to claim 2, which contains as active ingredient the compound of the formula

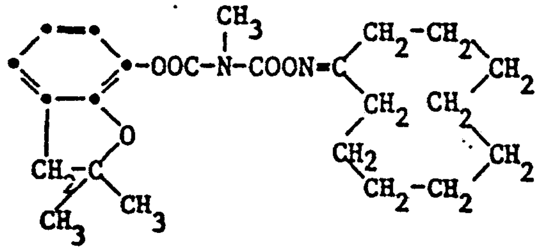

15. A composition according to claim 2, which contains as active ingredient the compound of the formula

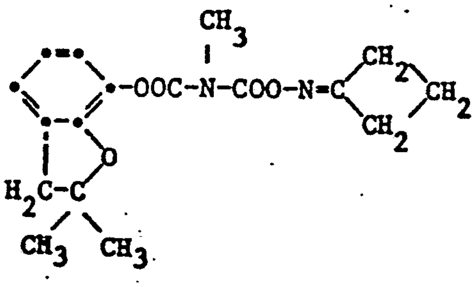

16. A process for the preparation of a compound of formula I according to claim 1, which process comprises reacting a compound of the formula

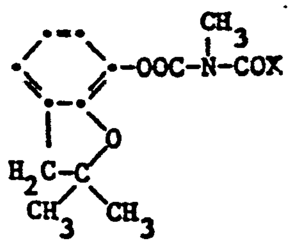

,

in the presence of a base, with a compound of the formula

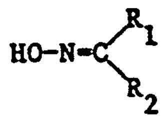

wherein $R_1$ and $R_2$ are as defined in claim 1, and X is a halogen atom.

17. Use of a composition according to claim 1 for controlling pests on animals and plants and in the soil.

18. Use according to claim 17 for controlling insects, and members of the order Acarina.

19. A method of controlling pests on animals and plants and in the soil, which method comprises applying to the animals, to the plants or to the soil an effective amount of a composition according to claim 1.

**Revendications**

pour les Etats contractants: BE, CH, DE, FR, IT, LI, NL.

1.- N-alcoylidène-imino-oxycarbonyl-N-méthyl-[2,2-diméthyl-2,3-dihydrobenzofuranyl(7)] -carbamates de formule

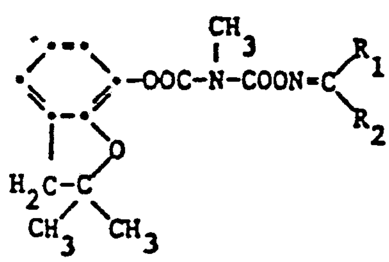

oü $R_1$ et $R_2$ représentent indépendamment l'un de l'autre un groupe alcoyle en $C_1$ à $C_{10}$ ou ensemble un groupe alcoylène en $C_2$ à $C_9$.

2.- Composé selon la revendication 1, oü $R_1$ et $R_2$ représentent indépendamment l'un de l'autre un groupe alcoyle en $C_1$ à $C_5$ ou ensemble un groupe alcoylène en $C_3$ à $C_9$.

3.- Composé selon la revendication 2, oü $R_1$ représente un groupe alcoyle en $C_1$ à $C_5$, $R_2$ un méthyle, éthyle ou propyle ou $R_1$ et $R_2$ représentent ensemble le groupe tétra- ou pentaméthylène.

4.- Le composé selon la revendication 3 de formule:

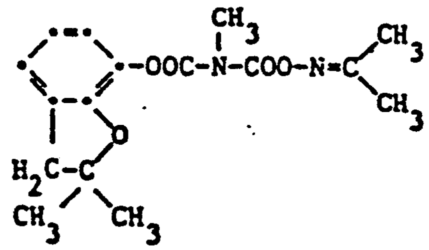

5. Le composé selon la revendication 3 de formule:

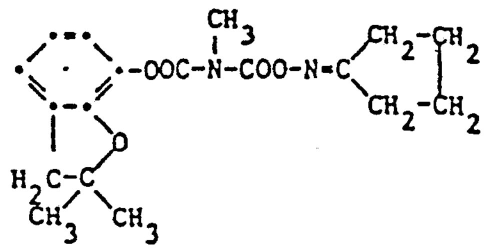

6. Le composé selon la revendication 3 de formule:

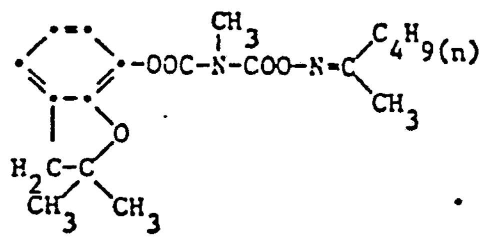

7. Le composé selon la revendication 3 de formule:

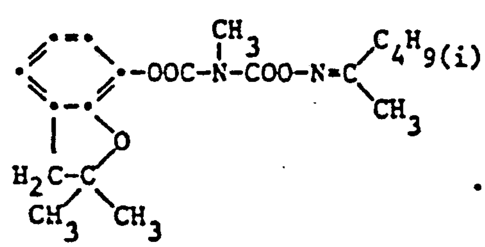

8. Le composé selon la revendication 3 de formule:

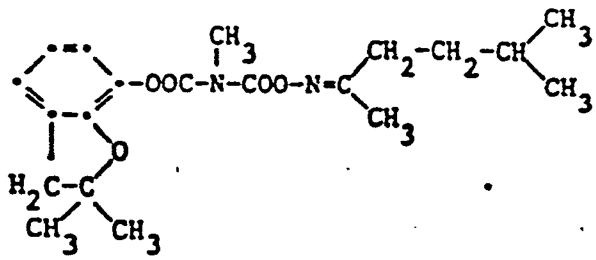

9. Le composé selon la revendication 3 de formule:

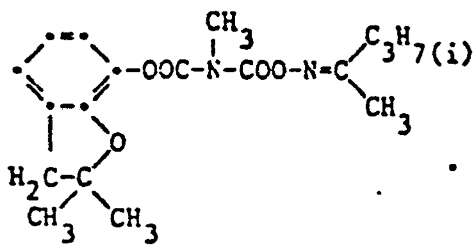

10. Le composé selon la revendication 3 de formule:

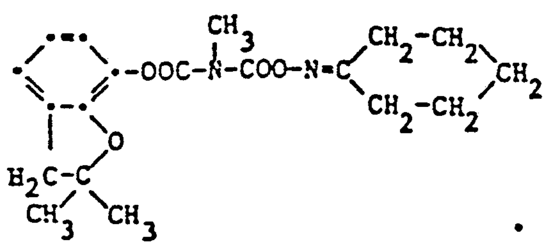

11. Le composé selon la revendication 1 de formule:

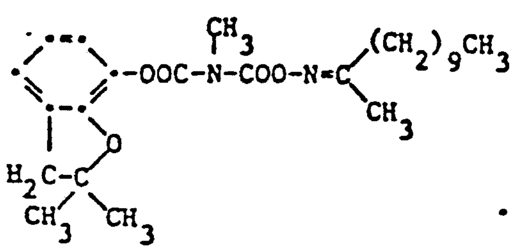

12. Le composé selon la revendication 3 de formule:

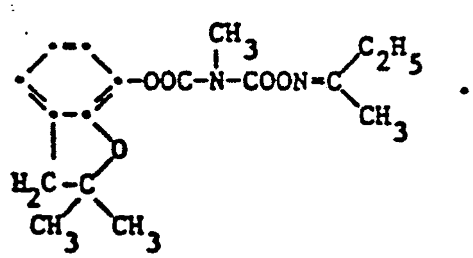

13. Le composé selon la revendication 3 de formule:

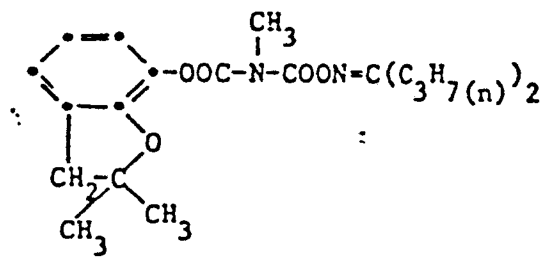

14. Le composé selon la revendication 2 de formule:

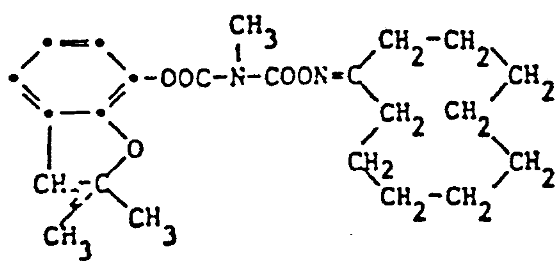

15. Le composé selon la revendication 2 de formule:

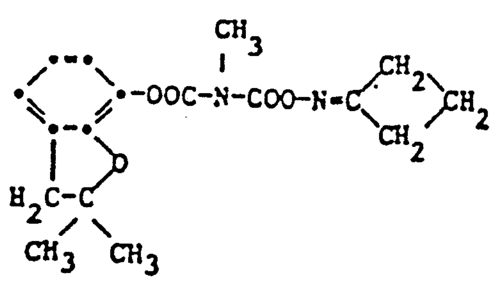

16.- Procédé de préparation de composés selon la revendication 1, caractérisé en ce qu'on fait réagir en présence d'une base un composé de formule:

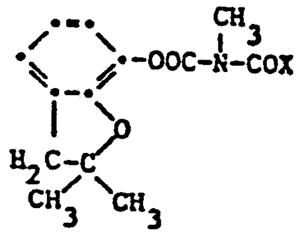

avec un composé de formule:

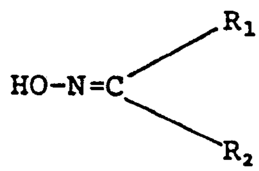

où $R_1$ et $R_2$ ont la signification donnée dans la revendication 1 et X représente un atome d'halogène.

17.- Agent de lutte antiparasitaire contenant comme composant actif un composé selon la revendication 1.

18.- Application d'un composé selon la revendication 1 à la lutte contre les parasites chez les animaux, les plantes et dans le sol.

19.- Application selon la revendication 18 à la lutte contre les insectes et les représentants de l'ordre des acariens.

20.- Procédé de lutte contre les parasites chez les animaux, les plantes et dans le sol, caractérisé en ce qu'on applique un composé selon la revendication 1 aux animaux, aux plantes ou au sol.

**Revendications**

pour l'Etat contractant: AT

1.- Agent de lutte antiparasitaire contenant outre un additif solide ou liquide, comme composant actif un N-alcoylidène-imino-oxycarbonyl-N-méthyl-[2,2-diméthyl-2,3-dihydrohenzofuranyl(7)]-carbamate de formule:

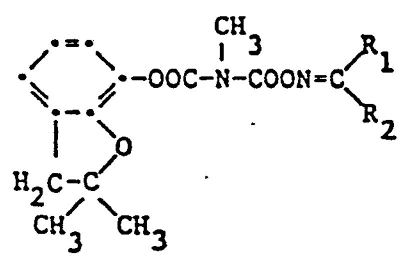

où $R_1$ et $R_2$ représentent indépendamment l'un de l'autre un groupe alcoyle en $C_1$ à $C_{10}$ ou ensemble un groupe alcoyléne en $C_2$ à $C_9$.

2.- Agent selon la revendication 1, où dans la formule du composant actif $R_1$ et $R_2$ représentent indépendamment l'un de l'autre un groupe alcoyle en $C_1$ à $C_5$ ou ensemble un groupe alcoyléne en $C_3$ à $C_9$.

3.- Agent selon la revendication 2, où dans la formule du composant actif $R_1$ représente un groupe alcoyle en $C_1$ à $C_5$, $R_2$ un méthyle, éthyle ou propyle ou $R_1$ et $R_2$ représentent ensemble le groupe tétra- ou pentaméthylène.

4.- Agent selon la revendication 3, contenant comme composant actif le composé de formule:

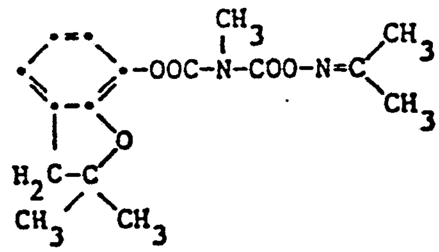

5.- Agent selon la revendication 3, contenant comme composant actif le composé de formule:

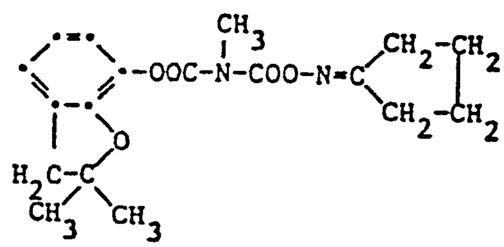

6.- Agent selon la revendication 3, contenant comme composant actif le composé de formule:

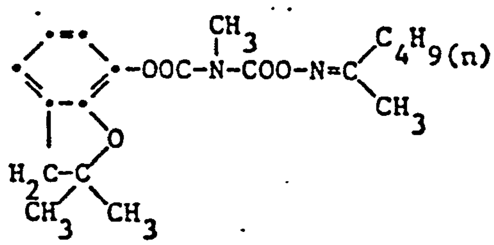

7.- Agent selon la revendication 3, contenant comme composant actif le composé de formule:

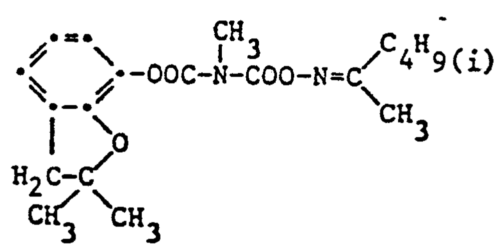

8.- Agent selon la revendication 3, contenant comme composant actif le composé de formule:

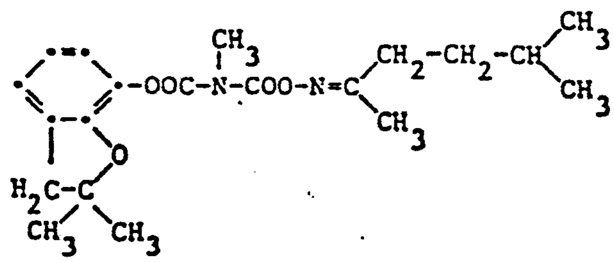

9.- Agent selon la revendication 3, contenant comme composant actif le composé de formule:

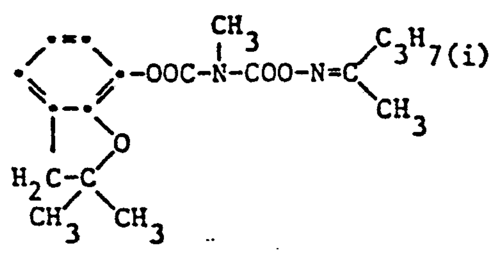

10.- Agent selon la revendication 3, contenant comme composant actif le composé de formule:

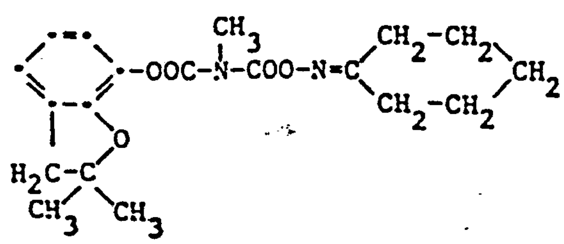

11.- Agent selon la revendication 1, contenant comme composant actif le composé de formule:

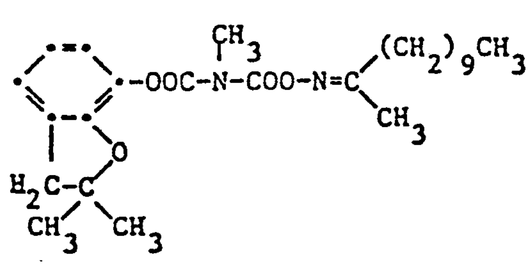

12.- Agent selon la revendication 3, contenant comme composant actif le composé de formule:

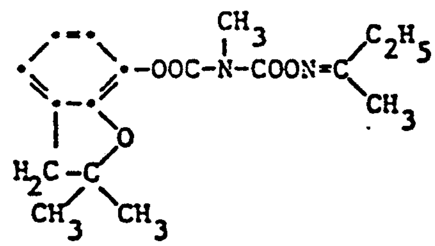

13.- Agent selon la revendication 3, contenant comme composant actif le composé de formule:

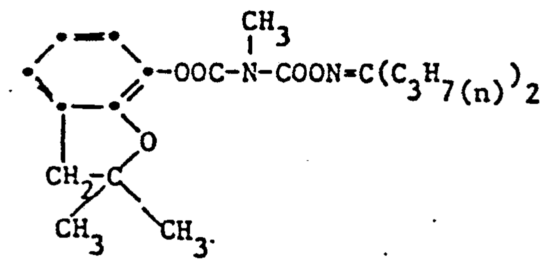

14.- Agent selon la revendication 2, contenant comme composant actif un composé de formule:

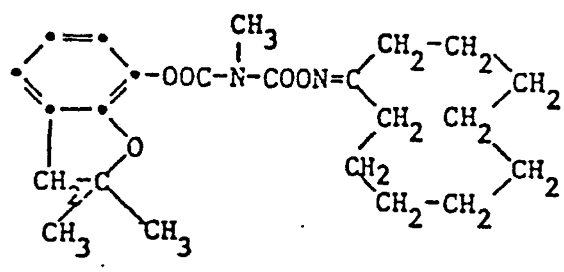

15.- Agent selon la revendication 2, contenant comme composant actif un composé de formule:

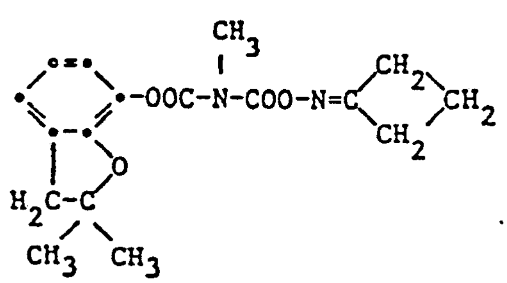

16.- Procédé de préparation de composés de formule I selon la revendication 1, caractérisé en ce qu'on fait réagir en présence d'une base un composé de formule:

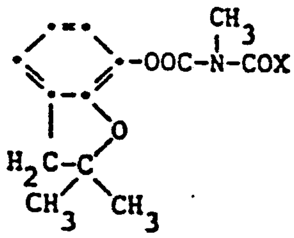

avec un composé de formule:

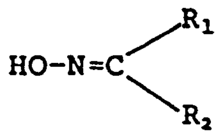

où $R_1$ et $R_2$ ont la signification donnée dans la revendication 1 et X représente un atome d'halogène.

17.- Application d'un agent selon la revendication 1 à la lutte contre les parasites chez les animaux, les plantes et dans le sol.

18.- Application selon la revendication 17 à la lutte contre les insectes et les représentants de l'ordre des acariens.

19.- Procédé de lutte contre les parasites chez les animaux, les plantes et dans le sol, caractérisé en ce qu'on applique un agent selon la revendication 1 aux animaux, aux plantes ou au sol.